# EUROPÄISCHE PATENTSCHRIFT

(11) **EP 1 408 825 B1**
(45) Veröffentlichungstag und Bekanntmachung des Hinweises auf die Patenterteilung: **12.01.2005**
(21) Anmeldenummer: 02760204.4
(22) Anmeldetag: 28.06.2002
(51) Int. Cl.: A61B 5/00, A61B 5/0448

(54) **SENSOREINRICHTUNG ZUM MESSEN VON VITALEN PARAMETERN EINES FETEN WÄHREND DER GEBURT**
SENSOR DEVICE FOR MEASURING VITAL PARAMETERS OF A FETUS DURING BIRTH
DISPOSITIF DE DETECTION SERVANT A MESURER DES PARAMETRES VITAUX D'UN FOETUS PENDANT LA NAISSANCE

(30) Priorität: 20.07.2001 DE 20112098 U
(43) Veröffentlichungstag der Anmeldung: 21.04.2004
(73) Patentinhaber: Rall, Gerhard, Dipl.-Ing., D-81545 München (DE); KNITZA, Reinhold, Dr., D-82131 Gauting (DE)
(72) Erfinder: Rall, Gerhard, Dipl.-Ing., D-81545 München (DE); KNITZA, Reinhold, Dr., D-82131 Gauting (DE)
(74) Vertreter: Grünecker, Kinkeldey, Stockmair & Schwanhäusser Anwaltssozietät
(86) Internationale Anmeldenummer: PCT/EP2002/007178
(87) Internationale Veröffentlichungsnummer: WO 2003/009747

(56) Entgegenhaltungen:
- EP-A- 0 611 548
- US-A- 4 281 659
- US-A- 5 012 811

## Beschreibung

Die Erfindung bezieht sich auf eine Sensoreinrichtung als Teil einer Messvorrichtung mit einem Messgerät, zum Messen von vitalen Parametern eines Feten während der Geburt, insbesondere des Sauerstoffgehalts des Blutes des Feten.

Eine aus der EP 611 548 A1 bekannte Sensoreinrichtung dieser Gattung weist einen runden schalenartigen Träger auf, in dem etwa zentral eine Befestigungszone vorgesehen ist, die mit einem spiralartigen Befestigungselement zum Befestigen des Trägers am führenden Teil des Feten versehen ist. Dabei ist die Drehachse des Befestigungselements etwa senkrecht zu der am führenden Teil des Feten anzubringenden Fläche des Trägers angeordnet. Der Träger weist weiter eine in Kontakt mit dem Gewebe des Feten bringbare Randzone auf, die einen Lichtemitter und einen Empfänger aufweist. Die elastische Randzone ist als Federmittel ausgebildet, wodurch sie unter federnder Vorspannung nachgiebig auf das Gewebe des Feten drückt. Dies gewährleistet ein zuverlässiges Übertragen von Licht von dem Emitter durch das Gewebe des Feten zu dem Empfänger, wobei gut verwertbare Signale erhalten werden.

Zum Anbringen der Sensoreinrichtung an dem Gewebe des Feten wird der Träger gegen das Gewebe des Feten gedrückt und die Sensoreinrichtung um die Achse und in Drehrichtung der Drahtspirale gedreht, so dass sie sich in die Kopfschwarte des Feten hineindreht. Die eingedrehte Drahtspirale gibt den Gegenhalt für die Federkraft, mit welcher die Randzone auf das Gewebe des Feten drückt.

Aus der US 5,199,432 und der US 5,373,843 ist es bekannt, einen zylindrischen CTG-Sensor (Kordiotokographie) an seiner Frontseite mit einer Drahtspirale zu versehen und durch Eindringen der Drahtspirale in das Gewebe eines Menschen daran zu befestigen. Zum Anbringen wird der zylinderförmige Sensor an seiner Rückseite mit einem Drehstab verbunden, der in einem Rohr drehbar gelagert ist. Die von dem Sensor abgehenden Leitungen erstrecken sich längs durch den Hohlraum des Drehstabs und werden rückseitig ausgeführt.

Beim Zuführen des Sensors ist der Sensor zunächst innerhalb des Rohres zurückgezogen angeordnet. Nach dem Aufsetzen des Rohres auf dem menschlichen Gewebe wird der Sensor mit Hilfe des Drehstabes axial nach vorne geschoben und dann verdreht. Es ergibt sich das Problem, dass die Handhabung der durch den Drehstab und das Rohr verlaufenden Leitungen in der Praxis umständlich ist, insbesondere wenn der Sensor an dem Gewebe angebracht ist und der Drehstab und das Rohr zurückgezogen werden, wenn sie nicht mehr gebraucht werden.

Eine analoge Befestigungsweise mit einer Drahtspirale wird in der US 4,644,957 vorgeschlagen, bei welcher sich dieselben Probleme ergeben. Bei derartigen Anordnungen ergibt sich zusätzlich der Nachteil, dass die Rohre häufig mit Ammnionflüssigkeit gefüllt werden, welche die Leitungen und Steckerkontakte beschmutzen. Wegen des Elektrolytgehalts der Ammnionflüssigkeit bleibt ein elektrisch leitfähiger Film zurück, der die Sensorsignale negativ beeinflussen kann.

Die US 6,058,321 beschreibt eine Messvorrichtung zum kontinuierlichen Überwachen eines fetalen Elektrokardiogramms und zum intermittierenden Überwachen eines Blut-pH-Wertes der fetalen Kopfhaut während einer Entbindung. An einer ebenen Unterseite der Vorrichtung ragt eine spiralförmige Nadel zum Eindrehen in die Kopfhaut hervor. Eine Hohlnadel der pH-Sonde ist während des Eindrehens zurückgezogen innerhalb der Vorrichtung gelagert, so dass sie keinen Kontakt mit dem Gewebe des Feten hat. Um das Basiselement ist ein drehbarer Haltering mit einer Aufnahmeöffnung für die Hohlnadel der pH-Sonde angeordnet, der beim Eindrehen der Spiralnadel an dem Basiselement arretiert ist. Wenn die Spiralnadel in der Kopfhaut des Feten sicher befestigt ist, kann die pH-Sonde mit dem Haltering in verschiedene beabstandete Messpositionen gedreht werden, von denen die Hohlnadel der pH-Sonde ausgefahren wird, um aus der fetalen Kopfhaut eine Blutprobe zu entnehmen.

Der Erfindung liegt die Aufgabe zugrunde, die bekannte Sensoreinrichtung der eingangs genannten Gattung so weiterzubilden, dass die dort vorhandenen Vorteile wie das sichere und lichtdichte am Gewebe des Feten erhalten bleiben, und darüber hinaus wenigstens der Träger (Sensor) schonender und leichter am Feten anzubringen ist.

Zur Lösung dieser Aufgabe sieht die Erfindung eine Sensoreinrichtung als Teil einer Messvorrichtung mit einem Messgerät, zum Messen von vitalen Parametern eines Feten während der Geburt, insbesondere des Sauerstoffgehalts des Blutes des Feten vor, wobei die Sensoreinrichtung einen schalenartigen Träger aufweist, der in eine etwa zentrisch angeordnete Befestigungszone und eine in Kontakt mit dem Gewebe des Feten bringbare Umgebungszone unterteilt ist, und die Befestigungszone mit einem spiralartigen Befestigungselement zum Befestigen des Trägers am führenden Teil des Feten versehen ist, wobei die Drehachse des Befestigungselements etwa senkrecht zu der am führenden Teil des Feten anzubringenden Fläche des Trägers angeordnet ist, und die Umgebungszone mindestens einen Lichtemitter und mindestens einen Empfänger aufweist, wobei das Befestigungselement gegenüber der Umgebungszone drehbar gelagert ist.

Durch die schalenartige Ausbildung des Trägers liegt die Umgebungszone schon beim Befestigen des spiralartigen Befestigungselementes am Gewebe des Feten auf und behält auch bei einem Eindrehen des Befestigungselementes in das Gewebe des Feten seine Position bei. Durch die erfindungsgemäße Maßnahme wird erreicht, dass beim Anbringen des Trägers am Gewebe des Feten nicht die bereits aufliegenden Teile des Träger unter Überwindung der Reibungskraft mit dem Befestigungselement mitgedreht werden müssen, das heißt das Anbringen des Trägers deutlich sensibler und gewebeschonender erfolgt.

Diese Handhabung des Trägers wird noch weiter erleichtert, wenn die Befestigungszone einen Drehkörper aufweist, an welchem das Befestigungselement befestigt ist.

Vorteilhaft ist dabei, wenn zu dem Messgerät signalübertragende Leitungen von der Umgebungszone abzweigend angeordnet sind. So können die Leitungen beim Eindrehen des Befestigungselements nicht hinderlich sein.

Gemäß einer bevorzugten Ausführungsform kann der Träger lösbar mit einer Drehhandhabe zum Zuführen und Anbringen der Sensoreinrichtung am führenden Teil des Feten verbindbar sein und die Leitungen des Trägers von dem vorderen Endbereich der Drehhandhabe nach außen abzweigend angeordnet sein. Somit können die Leitungen schon von dem vorderen Ende der Drehhandhabe abgeführt werden und im weiteren Verlauf so verlegt angeordnet werden, wie es für den Anwender am praktischsten ist. Insbesondere sind die Leitungen damit von dem größten Teil der Drehhandhabe befreit und werden von der Drehbewegung wenig beeinflusst.

Gemäß einer besonderen Ausgestaltung der Erfindung kann die Drehhandhabe eine Schutzglocke zum Beherbergen des Trägers und die Glockenwand eine Öffnung aufweisen, durch welche Leitungen nach außen geführt sind. Der Träger kann beim Zuführen und Anbringen des Sensors in der Schutzglocke angeordnet werden, durch maternales Gewebes von dem Träger ferngehalten und geschützt wird. Dies ermöglicht ein störungsfreies Anbringen des Trägers an dem Gewebe. Durch die Öffnung der Glockenwand werden Leitungen nach außen geführt und können somit nach dem Bedarf des Anwenders verlegt werden.

Es wird vorgeschlagen, dass die Öffnung zum Abführen der Leitungen in die dem führenden Teil des Feten zuzuwendende Trichteröffnung der Schutzglocke mündet. Beim Lösen der Drehhandhabe von dem am Feten angebrachten Träger werden die Leitungen über die Mündung der Öffnung der Glockenwand zur Trichteröffnung hin von der Schutzglocke gelöst und können je nach Einsatzbedarf angeordnet werden.

Gemäß einer speziellen Ausführungsform der Erfindung kann die Drehhandhabe einen mit der Befestigungszone verbindbaren Drehstab aufweisen, welcher drehbar in einer rohrartigen Hülle gelagert ist, wobei die Leitungen außerhalb der rohrartigen Hülle verlaufen. Während im Stand der Technik die Leitungen innerhalb der Hülle und dem Drehstab verliefen und beim Drehen des Drehstabs ein unerwünschtes Verdrehen der Leitungen bis hin zu einem Festsitz der Leitungen bzw. des Drehstabs in der Hülle auftreten konnte, ist bei dieser speziellen Ausführungsform der Erfindung ein ungestörtes Verdrehen des Drehstabes in der Hülle möglich. Die Leitungen können außerhalb der Hülle verlegt werden. Zum Beispiel ist ein Anbinden oder Anklipsen außen an die Hülle denkbar, wobei diese Verbindung gegebenenfalls auch wieder lösbar ist.

In besonderer Weise kann das Befestigungselement in elektrisch leitendem Kontakt mit einem Schleifringkontakt der Umgebungszone stehen. Dies ermöglicht, dass das Befestigungselement trotz seiner Drehbarkeit gegenüber der Umgebungszone als Elektrode verwendet werden kann, die ihr elektrisches Potential über den Schleifringkontakt an die Umgebungszone ableitet, von wo es über Leitungen weitergeführt wird. Somit lässt sich beispielsweise eine Drahtspirale als CTG-Elektrode verwenden.

In besonderer Weise kann die Befestigungszone über einen in einer Ringnut gelagerten Ringabsatz an dem Träger drehbar gelagert und axial festgelegt sein. Dies gewährleistet eine gute Verdrehbarkeit der Befestigungszone an dem Träger, wobei auch Andruckkräfte in Richtung der Drehachse auf den Träger übertragen werden. Dies ist besonders günstig bei Sensoren, deren Umgebungszone unter federnder Vorspannung auf das Gewebe des Feten drückt, um einen guten lichtübertragenden Kontakt und zu dem Gewebe des Feten herzustellen.

Besonders vorteilhaft kann die Befestigungszone axial an dem Träger verrastet sein. Über die Rastverbindung kann die zunächst separat hergestellte Befestigungszone an dem Träger befestigt werden, wobei die Rastverbindung ausreichend stabil für die zwischen Befestigungszone und Träger zu übertragenden Kräfte ist.

Ein Ausführungsbeispiel der Erfindung ist in der Zeichnung dargestellt und wird nachstehend erläutert. Es zeigen:
- Figur 1: eine gerade Draufsicht auf eine erfindungsgemäße Sensoreinrichtung,
- Figur 2: einen Vertikalschnitt durch die erfindungsgemäße Sensorvorrichtung von Figur 1 längs der Schnittlinie II-II in Figur 1,
- Figur 3: einen Teilschnitt durch die erfindungsgemäße Sensoreinrichtung gemäß Figur 1 längs der Schnittlinie III-III in Figur 1 und
- Figur 4: eine Seitenansicht einer erfindungsgemäßen Sensoreinrichtung als Teil einer Messvorrichtung mit einem Messgerät, in einer Situation kurz vor dem Anbringen an dem führenden Teil eines Feten.

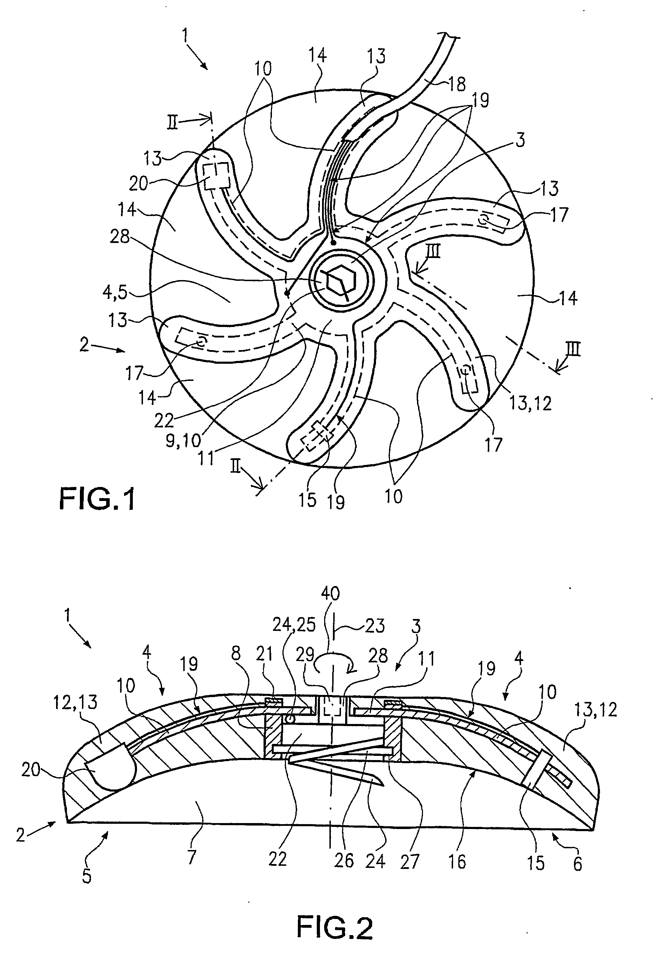

In den Figuren 1 und 2 ist eine erfindungsgemäße Sensoreinrichtung zum Messen von vitalen Parametern eines Feten während der Geburt dargestellt. Solche Sensoreinrichtungen werden auch als Pulsoxymetriesensoren bezeichnet.

Die Sensoreinrichtung hat einen in der Draufsicht etwa runden Träger 2, welcher in eine Befestigungszone 3 und eine elastische Umgebungszone 4 unterteilt ist. Die Befestigungszone 3 ist etwa zentral in dem Träger angeordnet, während die Umgebungszone 4 als Randzone ausgebildet ist und die Befestigungszone 3 umgibt.

Der Träger 2 ist als eine im Querschnitt gekrümmte runde Schale 5 ausgebildet. Sie weist auf ihrer dem führenden Teil eines Feten zuzuwendenden konkaven Seite 6 eine Höhlung 7 auf.

Die Umgebungszone 4 hat einen etwa zentral angeordneten Tragring 8, der aus hartem Kunststoff besteht. In den Tragring 8 ist ein Federstern 9 eingegossen, der sechs über den Träger verteilte radial nach außen abstehende Federarme 10 aufweist. Die Federarme 10 sind in der Draufsicht gesehen etwas im Uhrzeigersinn gebogen. Sie sind einstückig mit einem zentralen Tellerfederring 11 des Federnsterns 9 ausgebildet. Der Federstern 9 besteht aus einem etwa blattförmigen Federmetall.

Der Federstern 9 ist in ein weiches Silikonmaterial eingebettet, welches einen Schalenmantel 12 der Umgebungszone bildet und etwa die runde Form des Trägers 2 bildet. Das Silikonmaterial hat eine Härte von etwa 5 Shore.

Die Federarme 10 sind in relativ dicken Schalenarmen 13 des Schalenmantels 12 eingebettet, welche die Federarme 10 über ihre gesamte Erstreckungslänge umhüllen.

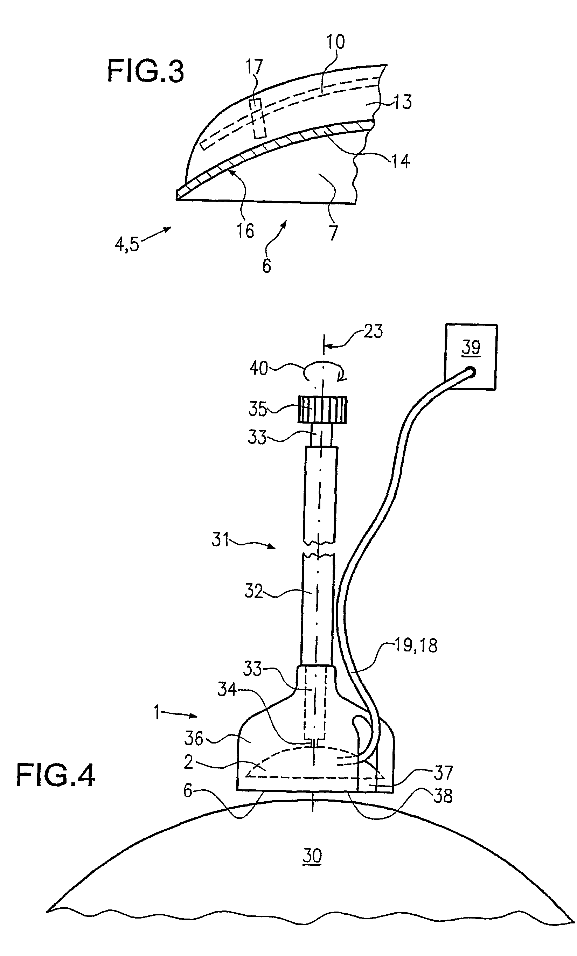

Zwischen den Schalenarmen 13 ist der Schalenmantel 12 als relativ dünne Schalenhaut 14 mit einer deutlich geringeren Wandstärke ausgebildet. In Figur 3 ist in einer Querschnittsansicht prinzipiell die dünne Schalenhaut 14 von in der Regel weniger als 1 mm Dicke und demgegenüber der um ein Vielfaches dickere Schalenarm 13 dargestellt, in welchem einer der Federarme 10 eingebettet ist.

Etwa am Endbereich eines der Federarme 10 ist ein Lichtemitter 15 angebracht, der mit der dem Feten zuzuwendenden Anlagefläche 16 fluchtet. Auf einem anderen Federarm 10 ist am Ende ein Lichtempfänger 20 angeordnet, der ebenfalls mit der Anlagefläche 16 bündig abschließt.

Drei andere Federarme 10 tragen nahe ihrem Endbereich einen etwa senkrecht zu ihrer Erstreckungsrichtung angeordneten Verstärkungssteg 17 aus hartem Kunststoff, der vollständig von Material des Schalenarmes 13 umgeben ist, wie in Figur 3 dargestellt.

An einem der Federarme 10 ist von außen ein Kabel 18 zugeführt, das sich in den zugehörigen Schalenarm 13 hineinerstreckt und an dem Federarm 10 befestigt ist. Das Kabel 18 erstreckt sich etwa in leicht tangentialer Richtung des Endes des Federarms. Innerhalb des Schalenmantels 12 erstrecken sich mehrere Leitungen 19 des Kabels 18. Jeweils eine Leitung ist zu dem Emitter 15 und dem Empfänger 20 gerichtet, wobei diese beiden Leitungen jeweils zweiadrig ausgebildet ist.

Eine der Leitungen 19 erstreckt sich zu dem Tellerfederring 11 und eine weitere Leitung 19 erstreckt sich zu einem metallischen Kontaktelement 21, welches an dem Tragring 8 angebracht ist. Über die Leitungen zu dem Kontaktelement 21 und dem Tellerfederring 11 können Signale für die Kardiotokographie abgeleitet werden.

Die Befestigungszone 3 weist einen Drehkörper 22 aus hartem Kunststoff auf, der innerhalb des Tragrings 8 drehbar gelagert ist. Die Drehachse 23 verläuft senkrecht zu der Anlagefläche 16 im Bereich des Drehkörpers 22. Die Drehrichtung ist mit dem Pfeil 40 angedeutet.

In den Drehkörper 22 ist eine Drahtspirale 24 eingebettet, die mit nicht ganz einer Windung zu der Seite 6 aus dem Drehkörper 22 herausragt. An der Seite 6 schließt der Drehkörper etwa bündig mit der Anlagefläche 16 ab.

Die Drahtspirale kann beispielsweise beim Herstellen des Drehkörpers 22 durch Spritzgießen mit eingegossen werden. Ein Windungsende 25 der Drahtspirale 24 ist in leitendem Kontakt mit dem Tellerfederring 11, so dass beim Verdrehen des Drehkörpers 22 mit der Drahtspirale 24 ein Schleifkontakt gebildet ist.

Der Drehkörper 22 weist umfangsseitig einen radial abstehenden Ringabsatz 26 auf, der in eine an der Innenseite des Tragrings 8 eingeformte Ringnut 27 eingreift und darin verdrehbar gelagert ist. Damit ist der Drehkörper 22 drehbar gelagert, aber axial festgelegt.

Zum Montieren des Drehkörpers 22 in dem Tragring 8 kann der Ringabsatz 26 in die Ringnut 27 eingerastet werden. Alternativ ist es denkbar, den Ringabsatz einseitig in axialer Richtung offen zu gestalten und nach dem Einsetzen des Drehkörpers 22 eine Nutwand an das offene Ende anzusetzen und z.B. durch Ultraschall an den Tragring 8 anzuschweißen. Wahlweise kann der Drehkörper 22 mit der Drahtspirale 24 als ein Wegwerfelement ausgebildet sein, das nach erfolgter Benutzung durch ein anderes ausgetauscht werden kann.

An der der Seite 6 abgewandten Seite hat der Drehabsatz einen verjüngten Abschnitt 28, in welchem zentral ein Innensechskant 29 ausgebildet ist. Der verjüngte Abschnitt 28 erstreckt sich durch die Schalendecke 12 hindurch und ist von außen zugänglich.

In Figur 4 ist die Situation beim Anbringen einer Sensoreinrichtung an dem führenden Teil eines Feten dargestellt, wobei exemplarisch der Kopf 30 des Feten angedeutet ist. Der Träger 2 ist an einer Drehhandhabe 31 angebracht. Sie weist ein äußeres begrenzt biegbares Rohr 32 auf, durch welches sich ein begrenzt biegbarer Drehstab 33 erstreckt. Der Drehstab 33 weist an seinem dem Kopf 30 zugewendeten Ende einen Sechskant 34 auf, der in den Innensechskant 29 eingreift. An der dem Feten abgewandten Seite ragt der Drehstab 33 aus dem Rohr 32 ein Stück weit hervor und hat an seinem Ende einen Drehkragen 35, der von einer Person per Hand verdreht werden kann. Der Drehstab 33 ist axial in dem Rohr 32 verschiebbar.

An dem dem Feten zugewandten Ende des Rohres 32 ist eine Schutzglocke 36 befestigt, in welcher der Träger 2 beherbergt ist. Das Kabel 18 des Trägers 2 zweigt im vorderen Bereich der Drehhandhabe 31 durch eine schlitzartige Öffnung 37 in der Wand der Schutzglocke 36 ab und ist dadurch nach außen geführt. Die Öffnung 37 mündet in die dem Feten zugewandte Trichteröffnung 38 der Schutzglocke 36. Das durch die Öffnung 37 nach außen geführte Kabel verläuft außerhalb des Rohres und ist an ein Messgerät 39 angeschlossen, das die erhaltenen Signale auswertet.

Im folgenden wird die Anwendung und Funktionsweise des in der Zeichnung dargestellten Ausführungsbeispiels einer erfindungsgemäßen Sensoreinrichtung näher erläutert.

Ausgehend von der in Figur 4 dargestellten Situation betätigt eine Person die Drehhandhabe 31 und führt damit die Sensoreinrichtung 1 mit dem Träger 2 innerhalb der Schutzglocke 36 auf den Kopf 30 des Feten zu. Die Schutzglocke 36 wird auf den Kopf 30 aufgesetzt und der Drehstab 33 axial nach vorne geschoben, so dass der Träger 2 auf den Kopf gedrückt wird. Dabei wird die Umgebungszone 4, die stärker gekrümmt ist als die statistisch durchschnittliche Krümmung des Kopfes des Feten, derart angedrückt, dass sie mit federnder Vorspannung auf dem Kopf aufliegt und als Federmittel dient. Der Lichtemitter 15 und der Empfänger 20 werden in Anlage gegen den Kopf 30 gedrückt.

In dem angedrückten Zustand wird der Drehstab 33 in Windungsrichtung der Drahtspirale 24 verdreht, so dass sie sich in die Kopfschwarte des Feten hineindreht. Dabei dreht sich nur der Drehkörper 22 mit der Drahtspirale 24. Die Umgebungszone 4 bleibt unverdreht in fester Anlage auf dem Kopf des Feten liegen. Das von der Umgebungszone 4 abzweigende Kabel 18 wird durch die Drehung nicht beeinflusst und erstreckt sich radial durch die Öffnung 37 nach außen.

Nachdem die Drahtspirale 24 festgedreht ist, wird der Drehstab 33 zurückgezogen, wobei er sich von dem Träger 2 löst. Dabei kann auch das Rohr mit der Schutzglocke zurückgezogen werden, so dass nur der Träger 2 an dem Kopf 30 verbleibt. Die Leitung ist durch die Verbindung der Öffnung 37 mit der Trichteröffnung 38 aus der Schutzglocke 36 ausführbar. Der an dem Kopf angebrachte Träger behält über die federnd vorgespannten Federarme 10 der Umgebungszone 4 seinen federnden Kontakt mit dem Kopf des Feten und es zweigt lediglich das Kabel 18 zu dem Messgerät 39 ab.

## Patentansprüche

1. Sensoreinrichtung (1) als Teil einer Messvorrichtung mit einem Messgerät (39), zum Messen von vitalen Parametern eines Feten (30) während der Geburt, insbesondere des Sauerstoffgehalts des Blutes des Feten, wobei die Sensoreinrichtung (1) einen schalenartigen Träger (2) aufweist, der in eine etwa zentrisch angeordnete Befestigungszone (3) und eine in Kontakt mit dem Gewebe des Feten (30) bringbare Umgebungszone (4) unterteilt ist, und die Befestigungszone (3) mit einem spiralartigen Befestigungselement (24) zum Befestigen des Trägers (2) am führenden Teil des Feten (30) versehen ist, wobei die Drehachse (23) des Befestigungselements (24) etwa senkrecht zu der am führenden Teil des Feten (30) anzubringenden Fläche (16) des Trägers (2) angeordnet ist, und die Umgebungszone (4) mindestens einen Lichtemitter (15) und mindestens einen Empfänger (20) aufweist, wobei das Befestigungselement (24) gegenüber der Umgebungszone (4) drehbar gelagert ist.

2. Sensoreinrichtung nach Anspruch 1,
**dadurch gekennzeichnet,**
**dass** die Befestigungszone (3) einen Drehkörper (22) aufweist, an welchem das Befestigungselement (24) befestigt ist.

3. Sensoreinrichtung nach wenigstens einem der vorhergehenden Ansprüche,
**dadurch gekennzeichnet,**
**dass** signalübertragende Leitungen (19) zu dem Messgerät (39) von der Umgebungszone (4) abzweigend angeordnet sind.

4. Sensoreinrichtung nach wenigstens einem der vorhergehenden Ansprüche,
**dadurch gekennzeichnet, dass** eine Drehhandhabe (31) vorgesehen ist, wobei der Träger (2) lösbar mit der Drehhandhabe (31) zum Zuführen und Anbringen der Sensoreinrichtung (1) am führenden Teil des Feten (30) verbindbar ist und Leitungen (19) des Trägers (2) von dem vorderen Endbereich der Drehhandhabe (31) nach außen abzweigend angeordnet sind.

5. Sensoreinrichtung nach Anspruch 4,
**dadurch gekennzeichnet,**
**dass** die Drehhandhabe (31) eine Schutzglocke (36) zum Beherbergen des Trägers (2) und die Glockenwand eine Öffnung (37) aufweist, durch welche Leitungen (19) nach außen geführt sind.

6. Sensoreinrichtung nach Anspruch 5,
**dadurch gekennzeichnet,**
**dass** die Öffnung (37) zum Abführen der Leitungen (19) in die dem führenden Teil des Feten (30) zuzuwendende Trichteröffnung (38) der Schutzglocke mündet.

7. Sensoreinrichtung nach wenigstens einem der Ansprüche 4 bis 6,
**dadurch gekennzeichnet,**
**dass** die Drehhandhabe (31) einen mit der Befestigungszone (3) verbindbaren Drehstab (33) aufweist, welcher drehbar in einer rohrartigen Hülle (32) gelagert ist, wobei die Leitungen (19) außerhalb der rohrartigen Hülle (32) verlaufen.

8. Sensoreinrichtung nach wenigstens einem der vorhergehenden Ansprüche,
**dadurch gekennzeichnet,**
**dass** das Befestigungselement (24) in elektrisch leitendem Kontakt mit einem Schleifringkontakt (11) der Umgebungszone (4) steht.

9. Sensoreinrichtung nach wenigstens einem der vorhergehenden Ansprüche,
**dadurch gekennzeichnet,**
**dass** die Befestigungszone (3) über einen in einer Ringnut (27) gelagerten Ringsabsatz (26) an dem Träger (2) drehbar gelagert und axial festgelegt ist.

10. Sensoreinrichtung nach wenigstens einem der vorhergehenden Ansprüche,
**dadurch gekennzeichnet,**
**dass** die Befestigungszone (3) axial an dem Träger (2) verrastet ist.

## Claims

1. Sensor device (1) as part of a measurement appliance with a measurement device (39) for the measurement of vital parameters of a foetus (30) during birth, in particular the oxygen content of the blood of the foetus, whereby the sensor device (1) exhibits a shell-type carrier (2), which is subdivided into an approximately centrally arranged attachment zone (3) and a surrounding zone (4), which can be brought into contact with the tissue of the foetus (30), and the attachment zone (3) is provided with a spiral-type attachment element (24) for attaching the carrier (2) to the leading part of the foetus (30), whereby the rotating axis (23) of the attachment element (24) is arranged approximately perpendicular to the surface (16) of the carrier (2) to be fitted to the leading part of the foetus (30), and the surrounding zone (4) exhibits at least one light emitter (15) and at least one receiver (20), whereby the attachment element (24) is supported for rotation with respect to the surrounding zone (4).

2. Sensor device according to claim 1,
**characterised in**
**that** the attachment zone (3) exhibits a rotating body (22) to which the attachment element (24) is attached.

3. Sensor device according to at least one of the preceding claims,
**characterised in**
**that** leads (19) transmitting the signal to the measurement device (39) are arranged branching off from the surrounding zone (4).

4. Sensor device according to at least one of the preceding claims,
**characterised in**
**that** a rotary handle (31) is provided, whereby the carrier (2) can be releasably connected to the rotary handle (31) for moving and fitting the sensor device (1) to the leading part of the foetus (30) and leads (19) of the carrier (2) are arranged branching off towards the outside from the front end section of the rotary handle (31 ).

5. Sensor device according to claim 4,
**characterised in**
**that** the rotary handle (31) exhibits a protective bell (36) for accommodating the carrier (2) and the bell wall exhibits an opening (37) through which leads (19) are brought out.

6. Sensor device according to claim 5,
**characterised in**
**that** the opening (37) for routing the leads (19) runs into the funnel opening (38) of the protective bell facing the leading part of the foetus (30).

7. Sensor device according to at least one of claims 4 to 6,
**characterised in**
**that** the rotary handle (31) exhibits a rotating bar (33) which can be connected to the attachment zone (3), said rotating bar being mounted for rotation in a tube-type sleeve (32), whereby the leads (19) run outside of the tube-type sleeve (32).

8. Sensor device according to at least one of the preceding claims,
**characterised in**
**that** the attachment element (24) is in electrically conducting contact with a slip-ring contact (11) of the surrounding zone (4).

9. Sensor device according to at least one of the preceding claims,
**characterised in**
**that** the attachment zone (3) is supported on the carrier (2) for rotation via an annular shoulder (26) supported in an annular groove (27) and is axially fixed.

10. Sensor device according to at least one of the preceding claims,
**characterised in**
**that** the attachment zone (3) is latched axially on the carrier (2).

## Revendications

1. Dispositif de détection (1) faisant partie d'un dispositif de mesure comportant un appareil de mesure (39) pour mesurer les paramètres vitaux d'un foetus (30) pendant l'accouchement, en particulier la teneur en oxygène du sang du foetus, étant précisé que le dispositif de détection (1) comporte un support en forme de calotte (2) qui est divisé en une zone de fixation (3) à peu près centrée et une zone environnante (4) apte à être mise en contact avec les tissus du foetus (30), que la zone de fixation (3) est pourvue d'un élément de fixation en spirale (24) pour la fixation du support (2) à la partie du foetus (30) qui se présente, que l'axe de rotation (23) de l'élément de fixation (24) est à peu près perpendiculaire à la surface (16) à placer sur la partie du foetus (30) qui se présente, que la zone environnante (4) comporte au moins un émetteur de lumière (15) et au moins un récepteur (20) et que l'élément de fixation (24) est monté en rotation par rapport à ladite zone environnante (4).

2. Dispositif de détection selon la revendication 1, **caractérisé en ce que** la zone de fixation (3) comporte un corps rotatif (22) auquel est fixé l'élément de fixation (24).

3. Dispositif de détection selon l'une au moins des revendications précédentes, **caractérisé en ce que** des lignes de transmission de signaux (19) partent de la zone environnante (4) pour être branchées à l'appareil de mesure (39).

4. Dispositif de détection selon l'une au moins des revendications précédentes, **caractérisé en ce qu'**il est prévu une manette tournante (31), le support (2) étant apte à être relié de manière amovible à ladite manette (31) pour amener et appliquer le dispositif de détection (1) sur la partie du foetus (30) qui se présente, et des lignes (19) du support (2) partent de la zone d'extrémité avant de la manette tournante (31) et s'étendent vers l'extérieur.

5. Dispositif de détection selon la revendication 4, **caractérisé en ce que** la manette tournante (31) comporte une cloche de protection (36) pour loger le support (2), et la paroi de la cloche présente une ouverture (37) à travers laquelle les lignes (19) s'étendent vers l'extérieur.

6. Dispositif de détection selon la revendication 5, **caractérisé en ce que** l'ouverture (37) destinée au passage des lignes (19) débouche dans l'ouverture en entonnoir (38) de la cloche de protection à tourner vers la partie du foetus (30) qui se présente.

7. Dispositif de détection selon l'une au moins des revendications 4 à 6, **caractérisé en ce que** la manette tournante (31) comporte une tige tournante (33) qui est apte à être reliée à la zone de fixation (3) et qui est montée en rotation dans une gaine tubulaire (32), les lignes (19) s'étendant à l'extérieur de la gaine tubulaire (32).

8. Dispositif de détection selon l'une au moins des revendications précédentes, **caractérisé en ce que** l'élément de fixation (24) est en contact conducteur avec un contact à bague glissante (11) de la zone environnante (4).

9. Dispositif de détection selon l'une au moins des revendications précédentes, **caractérisé en ce que** la zone de fixation (3) est montée en rotation et fixée axialement au support (2) par l'intermédiaire d'un épaulement annulaire (26) monté dans une rainure annulaire (27).

10. Dispositif de détection selon l'une au moins des revendications précédentes, **caractérisé en ce que** la zone de fixation (3) est enclenchée axialement sur le support (2).
